# EUROPEAN PATENT APPLICATION

(11) **EP 4 491 736 A1**
(43) Date of publication of application: **15.01.2025**
(21) Application number: 22933753.0
(22) Date of filing: 27.07.2022
(51) Int. Cl.: C12P 23/00, C12N 15/81, C12N 1/16, C12N 1/38, C12R 1/645

(54) **MICROBIAL MEDIUM COMPOSITION FOR PRODUCING RETINOL, COMPRISING ANTI-OXIDANT, AND USE THEREOF**

(30) Priority: 23.03.2022 KR 20220036256
(71) Applicant: CJ CheilJedang Corporation, Seoul 04560 (KR)
(72) Inventor: PARK, Hye Min, Seoul 04560 (KR); LEE, Peter, Seoul 04560 (KR); LEE, Dong Pil, Seoul 04560 (KR); KIM, Jae Eung, Seoul 04560 (KR)
(74) Representative: Meissner Bolte Partnerschaft mbB
(86) International application number: PCT/KR2022/011046
(87) International publication number: WO 2023/182581

(57) **Abstract**

Provided are a method of producing retinol, the method comprising the step of culturing a microorganism of the genus *Yarrowia* in a medium containing an antioxidant; a method of increasing retinol production; a method of producing retinoids; a medium composition for a microorganism of the genus *Yarrowia* for producing retinol, the composition comprising an antioxidant; a composition for producing retinol, the composition comprising the microorganism or a culture thereof and an antioxidant.

## Description

### [Technical Field]

The present disclosure relates to a method of producing retinol, the method comprising the step of culturing a microorganism of the genus *Yarrowia* in a medium containing an antioxidant; a method of increasing retinol production; a method of producing retinoids; a medium composition for a microorganism of the genus *Yarrowia* for producing retinol, the composition comprising an antioxidant; a composition for producing retinol, the composition comprising the microorganism or a culture thereof and an antioxidant; and use thereof.

### [Background Art]

Retinol, which is a fat-soluble vitamin, is an essential vitamin involved in eye health of improving nyctalopia, immune enhancement, skin health, etc. However, since retinol is very unstable to heat, light, temperature, moisture, oxygen, and progress of time, it is easily oxidized when exposed to the air or in aqueous solutions. This causes the lower potency of raw materials, major stability issues such as discoloration, off-smell, etc., and also a negative impact on retinol production.

Accordingly, many technologies have been developed to stabilize the retinol compound itself in compositions or products containing retinol (US Patent No. 6858217). However, the development of methods of stably increasing retinol production remains insignificant.

### [Disclosure]

### [Technical Problem]

The problem to be solved in the present disclosure is to provide a microorganism medium composition for producing retinol, the composition comprising an antioxidant, a method of producing retinoids using the same, and use thereof.

### [Technical Solution]

An object of the present disclosure is to provide a method of producing retinol using an antioxidant.

Another object of the present disclosure is to provide a method of increasing retinol production using an antioxidant.

Still another object of the present disclosure is to provide a method of producing retinoids other than retinol using an antioxidant.

Still another object of the present disclosure is to provide a microorganism medium composition for producing retinol using an antioxidant.

Still another object of the present disclosure is to provide a composition for producing retinol.

Still another object of the present disclosure is to provide use of an antioxidant in producing retinoids.

### [Advantageous Effects]

A medium of the present disclosure may efficiently increase production of retinoids such as retinol by comprising an antioxidant.

### [Brief Description of the Drawing]

FIG. 1 shows a microorganism growth rate according to a culture time in a medium to which an antioxidant is added.
FIG. 2 shows an increase in retinol production due to the addition of the antioxidant.

### [Detailed Description of Preferred Embodiments]

Hereinafter, the present disclosure will be described in detail as follows. Meanwhile, each description and embodiment disclosed in this disclosure may also be applied to other descriptions and embodiments. Further, all combinations of various elements disclosed in this disclosure fall within the scope of the present disclosure. Furthermore, literatures described in the present disclosure are incorporated herein by reference. Further, the scope of the present disclosure is not limited by the specific description described below.

An aspect of the present disclosure provides a method of producing retinol, the method comprising the step of culturing a microorganism of the genus *Yarrowia* in a medium containing an antioxidant.

As used herein, the term "antioxidant" collectively refers to a substance that prevents oxidation. The antioxidation may be used interchangeably with prevention of oxidative stress, prevention of reactive oxygen, and/or anti-aging, but the antioxidation is not limited thereto as long as it may prevent oxidation and may increase retinol production.

The antioxidant may be any one or more selected from the group consisting of 3,5-di-tert-4-butylhydroxytoluene (BHT), propyl gallate (PG), vitamin C (ascorbic acid), and glutathione (GSH), but is not limited thereto.

The antioxidant may be included at a concentration of 0.0001 %(w/v) to 10%(w/v), 0.0001%(w/v) to 5%(w/v), 0.0001%(w/v) to 1%(w/v), 0.001%(w/v) to 10%(w/v), 0.001%(w/v) to 5%(w/v), 0.001%(w/v) to 1%(w/v), 0.01%(w/v) to 10%(w/v), 0.01%(w/v) to 5%(w/v), 0.01%(w/v) to 1%(w/v), 0.01%(w/v) to 0.09%(w/v), 0.01%(w/v) to 0.08%(w/v), 0.01%(w/v) to 0.07%(w/v), 0.01%(w/v) to 0.06%(w/v), or 0.01%(w/v) to 0.05%(w/v) with respect to the total medium composition, but is not limited thereto.

In one embodiment, retinol may be stably produced while minimizing the consumption of time and labor resources by comprising the step of culturing the microorganism of the genus *Yarrowia* in the medium containing the antioxidant.

As used herein, the term the "medium" refers to a mixture containing, as main ingredients, nutrient materials required for culturing the microorganism of the genus *Yarrowia* of the present disclosure, wherein the medium supplies nutrient materials comprising water essential for survival and growth, growth factors, etc.

In one embodiment, the medium of the present disclosure may be a medium for producing retinol, and may further comprise substances required for retinol production, but is not limited thereto.

As for the media and other culture conditions used for culturing the microorganism of the genus *Yarrowia* of the present disclosure, any common medium already containing an antioxidant or any medium used for usual culture of microorganisms while further containing the antioxidant may be used without particular limitation.

The medium of the present disclosure may be a common medium containing appropriate carbon sources, nitrogen sources, phosphorus sources, inorganic compounds, amino acids, and/or vitamins, while controlling the temperature, pH, etc., but is not limited thereto.

In the present disclosure, the carbon sources may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of other carbon sources may be variously used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphate sources may comprise potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be included. These constituent ingredients or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, sulfuric acid, etc., to the medium in an appropriate manner during the culture of the microorganism of the genus *Yarrowia* of the present disclosure. In addition, an anti-foaming agent, such as fatty acid polyglycol ester, may be used to suppress bubble formation during the culture. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state, but is not limited thereto.

As used herein, the term "microorganism" or "strain" includes all of wild-type microorganisms or naturally or artificially genetically modified microorganisms, and it may also include a microorganism comprising genetic modification for retinol production, which is a microorganism in which a specific mechanism is weakened or strengthened due to insertion of a foreign gene or an activity enhancement or inactivation of an endogenous gene.

In one embodiment, the microorganism of the present disclosure may be a microorganism of the genus *Yarrowia* (*Yarrowia* sp.), but is not limited thereto.

In one embodiment, the microorganism of the present disclosure may be *Yarrowia lipolytica,* but is not limited thereto.

In one embodiment, the microorganism of the present disclosure may be a microorganism for producing retinol. The microorganism or strain for producing retinol may be a microorganism naturally having a retinol producing ability, or a microorganism in which the retinol producing ability is enhanced or provided due to natural or artificial genetic modification in a parent strain having no retinol producing ability, but is not limited thereto. Specifically, the microorganism for producing retinol of the present disclosure may be a microorganism of the genus *Yarrowia,* which is modified to comprise polynucleotides encoding lycopene cyclase/phytoene synthase (crtYB), phytoene desaturase (crtl), and beta-carotene 15, 15'-oxygenase (BLH) proteins.

The microorganism of the present disclosure may be a microorganism which is modified to further comprise polynucleotides encoding lycopene cyclase/phytoene synthase (crtYB) and phytoene desaturase (crtl) proteins, thereby exhibiting activities of the proteins, or a microorganism in which the activities of the proteins are enhanced. The lycopene cyclase/phytoene synthase, or phytoene desaturase may be a protein derived from *Xanthophyllomyces dendrorhous*, but is not limited thereto. In one embodiment, the polynucleotide encoding the lycopene cyclase/phytoene synthase or phytoene desaturase may have or comprise a nucleotide sequence based on GenBank: AY177204.1 or GenBank: AY177424.1 which is registered in National Center for Biotechnology Information Search database (NCBI), respectively. In one embodiment, the polynucleotide encoding the lycopene cyclase/phytoene synthase or phytoene desaturase may have or comprise SEQ ID NO: 1 or 2, respectively. The polynucleotide may undergo various modifications in the coding region within the scope that does not change the amino acid sequence in consideration of codon degeneracy or codons preferred in microorganisms that are intended to express the protein. Specifically, the polynucleotide may have or comprise a nucleotide sequence having 80% or more, 85% or more, 90% or more, 95% or more, 96% or more, 97% or more, 98% or more, and less than 100% homology or identity to the sequence of SEQ ID NO: 1 or SEQ ID NO: 2, or may consist of or essentially consist of a nucleotide sequence having 80% or more, 85% or more, 90% or more, 95% or more, 96% or more, 97% or more, 98% or more, and less than 100% homology or identity to the sequence of SEQ ID NO: 1 or SEQ ID NO: 2, but is not limited thereto.

The microorganism of the present disclosure may be a microorganism which is modified to further comprise a polynucleotide encoding geranylgeranyl pyrophosphate synthase (GGPPS) protein, thereby exhibiting activity of the protein, or a microorganism in which the activity of the protein is enhanced, but is not limited thereto. The geranylgeranyl pyrophosphate synthase may be a protein derived from *Haematococcus pluvialis*, but is not limited thereto. In one embodiment, the polynucleotide encoding the geranylgeranyl pyrophosphate synthase may have or comprise a nucleotide sequence based on GenBank: APX64485.1 which is registered in National Center for Biotechnology Information Search database (NCBI). In one embodiment, the polynucleotide encoding the geranylgeranyl pyrophosphate synthase may have or comprise a sequence of SEQ ID NO: 33. The polynucleotide may undergo various modifications in the coding region within the scope that does not change the amino acid sequence in consideration of codon degeneracy or codons preferred in microorganisms that are intended to express the protein. Specifically, the polynucleotide may have or comprise a nucleotide sequence having 80% or more, 85% or more, 90% or more, 95% or more, 96% or more, 97% or more, 98% or more, and less than 100% homology or identity to the sequence of SEQ ID NO: 33, or may consist of or essentially consist of a nucleotide sequence having 80% or more, 85% or more, 90% or more, 95% or more, 96% or more, 97% or more, 98% or more, and less than 100% homology or identity to the sequence of SEQ ID NO: 33, but is not limited thereto.

The microorganism of the present disclosure may be a microorganism which is modified to further comprise a polynucleotide encoding beta-carotene 15, 15'-oxygenase (BLH) protein, thereby exhibiting activity of the protein, or a microorganism in which the activity of the protein is enhanced, but is not limited thereto. The beta-carotene 15, 15'-oxygenase may be a protein derived from *Uncultured marine bacterium* 66A03, but is not limited thereto. In one embodiment, the beta-carotene 15, 15'-oxygenase polypeptide and a polynucleotide encoding the same may have or comprise an amino acid sequence based on Q4PNI0 which is registered in UniProt Knowledgebase (UniProtKB). In one embodiment, the beta-carotene 15, 15'-oxygenase polypeptide may have or comprise a sequence of SEQ ID NO: 57. The polynucleotide may undergo various modifications in the coding region within the scope that does not change the amino acid sequence in consideration of codon degeneracy or codons preferred in microorganisms that are intended to express the protein. Specifically, the polynucleotide may have or comprise a nucleotide sequence having 80% or more, 85% or more, 90% or more, 95% or more, 96% or more, 97% or more, 98% or more, and less than 100% homology or identity to the sequence of SEQ ID NO: 57, or may consist of or essentially consist of a nucleotide sequence having 80% or more, 85% or more, 90% or more, 95% or more, 96% or more, 97% or more, 98% or more, and less than 100% homology or identity to the sequence of SEQ ID NO: 57, but is not limited thereto.

As used herein, the term "culture" refers to growing the microorganism of the genus *Yarrowia* of the present disclosure in appropriately adjusted environment conditions. In the present disclosure, as long as the medium containing the antioxidant is used, the culture procedure may be performed according to appropriate media or culture conditions known in the art. Such a culture procedure may be easily adjusted according to the selected strain by a person skilled in the art. Specifically, the culture may be in a batch type, a continuous type, and/or a fed-batch type, but is not limited thereto.

The microorganism of the genus *Yarrowia* of the present disclosure may be cultured under aerobic conditions in a common medium containing appropriate carbon sources, nitrogen sources, phosphorus sources, inorganic compounds, amino acids and/or vitamins, etc. while controlling temperature, pH, etc.

In the culture of the present disclosure, the culture temperature may be maintained at 20°C to 35°C, specifically, at 25°C to 35°C, and the culture may be performed for about 10 hours to 160 hours, about 20 hours to 130 hours, about 24 hours to 120 hours, about 36 hours to 120 hours, about 48 hours to 120 hours, about 48 hours, about 72 hours, or about 120 hours, but is not limited thereto.

The retinol which is produced by the culture of the present disclosure may be released into the medium or may remain within the microorganism.

As used herein, the term "retinol" is a substance known as vitamin A and is a kind of retinoids. The retinol may be used as it is, but may be converted to other retinoids (e.g., retinal, retinoic acid, and retinyl esters, etc.) or carotenoid compounds by methods known in the art.

In the present disclosure, the antioxidant may be added to the microorganism medium for producing retinol to remarkably increase the retinol-producing ability.

For example, when the microorganism is cultured in a medium to which the antioxidant is added, it may have about 1% or more, specifically, about 3%, about 5% or more, about 10% or more, about 20% or more, about 30% or more, about 40% or more, about 50% or more, about 60% or more, about 70% or more, or about 80% or more increased retinol-producing ability, as compared to that of the microorganism cultured in a medium to which the antioxidant is not added. However, as long as the microorganism has an increased ability of + value, as compared to that before addition of the antioxidant, it is not limited thereto.

As used herein, the term "about" refers to a range which includes all of ±0.5, ±0.4, ±0.3, ±0.2, ±0.1, etc., and includes all of the values that are equivalent or similar to those following the term "about", but the range is not limited thereto.

The method of producing retinol of the present disclosure may further comprise the steps of preparing the microorganism of the genus *Yarrowia* of the present disclosure, preparing a medium for culturing the microorganism, or a combination of these steps (regardless of the order, in any order), for example, before or after the culturing step.

The method of producing retinol of the present disclosure may further comprise the step of recovering retinol from the medium resulting from the culture (a medium in which culture has been performed) or from the microorganism of the present disclosure. The recovering step may be further included after the culturing step.

The recovering may be collecting the desired retinol by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, cell disruption, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and retinol may be recovered from the medium or microorganism by using an appropriate method known in the art.

In addition, the method of producing retinol of the present disclosure may further comprise a purification step. The purification may be performed by using an appropriate method known in the art. In an exemplary embodiment, when the method of producing retinol of the present disclosure comprises both the recovering step and the purification step, the recovering step and the purification step may be performed discontinuously (or continuously) regardless of the order, or may be performed simultaneously or integrated into one step, but is not limited thereto.

Another aspect of the present disclosure provides a method of producing retinoids, the method comprising the steps of culturing the microorganism of the genus *Yarrowia* in the medium containing the antioxidant; and converting retinol which is produced by the microorganism, into retinoids other than retinol.

The antioxidant, medium, microorganism, culture, retinol, and retinoids are as described in other aspects, and the above-described retinol recovery and purification may also be equally applied to retinoid recovery and purification.

The method of producing retinoids of the present disclosure may further comprise the step of converting retinol which is produced by the microorganism of the present disclosure, into retinoids other than retinol. In the method of producing retinoids of the present disclosure, the converting step may be further included after the culturing step or the recovering step. The converting step may be performed using a suitable method known in the art. For example, the converting may be performed using retinol acyltransferase, but is not limited thereto.

In one embodiment, the retinoid may be any one selected from the group consisting of retinol, retinal, retinoic acid, and retinyl ester, but is not limited thereto, as long as it is included in the retinoids.

Still another aspect of the present disclosure provides a method of increasing retinol production, the method comprising the step of culturing the microorganism of the genus *Yarrowia* in the medium containing the antioxidant.

The antioxidant, medium, microorganism, culture, and retinol are as described in other aspects.

Still another aspect of the present disclosure provides a medium composition for the microorganism of the genus *Yarrowia* for producing retinol, the composition comprising the antioxidant.

In one embodiment, the medium composition may increase retinol production of the microorganism of the genus *Yarrowia,* but is not limited thereto.

In one embodiment, the medium composition may increase growth of the microorganism, but is not limited thereto.

The antioxidant, retinol, microorganism, and medium are as described in other aspects.

Still another aspect of the present disclosure provides a composition for producing retinol, the composition comprising the microorganism of the genus *Yarrowia* or the culture thereof, and the antioxidant.

The composition of the present disclosure may further comprise any appropriate excipient that is usually used in the composition for producing retinol, and examples of the excipient may comprise a preserving agent, a wetting agent, a dispersing agent, a suspending agent, a buffering agent, a stabilizing agent, an isotonic agent, etc., but are not limited thereto.

The microorganism, antioxidant, and retinol are as described in other aspects.

Still another aspect of the present disclosure provides use of the antioxidant in producing retinol; use of the medium composition for the microorganism of the genus *Yarrowia,* the composition comprising the antioxidant, in producing retinol; and use of the composition comprising the microorganism of the genus *Yarrowia* or the culture thereof and the antioxidant in producing retinoids. With regard to the use in producing retinoids, the retinoids may be retinol or retinoids other than retinol.

The antioxidant, microorganism, medium, retinoids, and retinol are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Preparation of Yarrowia lipolytica platform strains for retinol production

### Example 1-1. Preparation of X. dendrorhous-derived crtYB-crtl inserted strain

To prepare *Yarrowia* platform strains for retinol production, lycopene cyclase/phytoene synthase (crtYB) and phytoene desaturase (crtl) genes derived from *Xanthophyllomyces dendrorhous* were inserted into the genome of the high-fat yeast KCCM12972P strain.

A polynucleotide of SEQ ID NO: 1 of crtYB was obtained, based on a nucleotide sequence (GenBank: AY177204.1) registered in the National Center for Biotechnology Information Search database (NCBI), and a polynucleotide of SEQ ID NO: 2 of crtl was obtained, based on a nucleotide sequence (GenBank: AY177424.1) registered in the NCBI. The polynucleotide sequences of crtYB and crtl were synthesized by Macrogen in the form of TEFINtp-crtYB-CYC1t (SEQ ID NO: 3), and TEFINtp-crtl-CYC1t (SEQ ID NO: 4), respectively. A cassette to be inserted into the MHY1 (YALI0B21582g) gene site was designed using a URA3 gene (SEQ ID NO: 5) of *Y*. *lipolytica* as a selection marker.

Each PCR was performed using the synthesized crtYB and crtl genes and KCCM12972P genomic DNA as templates, and primers of SEQ ID NOS: 6 and 7, SEQ ID NOS: 8 and 9, SEQ ID NOS: 10 and 11, SEQ ID NOS: 12 and 13, SEQ ID NOS: 14 and 15, and SEQ ID NOS: 16 and 17, as shown in Table 1. PCR was performed by 35 cycles consisting of denaturation at 95°C for 1 min; annealing at 55°C for 1 min; and polymerization reaction at 72°C for 3 min. The resulting DNA fragments were prepared as a single cassette through overlap extension PCR.

The cassette thus prepared was introduced into KCCM12972P strain by a heat shock method *(*D.-C. Chen et al., Appl Microbiol Biotechnol, 1997), and then colonies were obtained, which were formed on a solid medium (YLMM1) without uracil. Colonies in which cassette insertion into the genome was confirmed using primers of SEQ ID NOS: 18 and 19 were plated on a 5-FOA solid medium and cultured at 30°C for 3 days, and colonies grown on the 5-FOA solid medium were obtained to recover the URA3 marker.

### <5-Fluoroorotic Acid (5-FOA)>

20 g/L of glucose, 6.7 g/L of yeast nitrogen base without amino acids, 2 g/L of yeast synthetic drop-out medium supplements without uracil, 50 µg/mL of uracil, 1 g/L of 5-fluoroorotic acid (5-FOA), 15 g/L of agar.

**[Table 1]**

| SEQ ID NO. | Sequence (5'-3') | PCR product |
|---|---|---|
| 6 | GTGCGCTTCTCTCGTCTCGGTAACCCTGTC | Homology left arm |
| 7 | ATGCGCCGCCAACCCGGTCTCTGGGGTGTGGTGGATGGGGTGTG | |
| 8 | CACACCCCATCCACCACACCCCAGAGACCGGGTTGGCGGCGCAT | TEFINtp-crtYB-CYC1t |
| 9 | CGCCGCCAACCCGGTCTCTTGAAGACGAAAGGGCCTCCG | |
| 10 | CGGAGGCCCTTTCGTCTTCAAGAGACCGGGTTGGCGGCG | TEFINtp-crtl-CYC1t |
| 11 | GACGAGTCAGACAGGAGGCATCAGACAGATACTCGTCGCG | |
| 12 | CGCGACGAGTATCTGTCTGATGCCTCCTGTCTGACTCGTC | URA3 |
| 13 | ATGACGAGTCAGACAG GAG GCATG GTG GTATTGTGACTGG GGAT | |
| 14 | ATCCCCAGTCACAATACCACCATGCCTCCTGTCTGACTCGTCAT | Repeat region |
| 15 | CGGCGTCCTTCTCGTAGTCCGCTTTTGGTGGTGAAGAGGAGACT | |
| 16 | AGTCTCCTCTTCACCACCAAAAGCGGACTACGAGAAGGACGCCG | Homology right arm |
| 17 | CCACTCGTCACCAACAGTGCCGTGTGTTGC | |
| 18 | TCGTACGTCTATACCAACAGATGG | Forward |
| 19 | CGCATACACACACACTGCCGGGGG | Reverse |

### Example 1-2. Preparation of HMGR-enhanced strain

A cassette for replacement of a native promoter (SEQ ID NO: 20) region of 3-hydroxy-3-methylglutaryl-CoA reductase (HMGR) gene of the strain which was prepared through Example 1-1 with a TEFINt promoter was designed, and each PCR was performed using genomic DNA of KCCM12972P as a template, and primers of SEQ ID NOS: 21 and 22, SEQ ID NOS: 23 and 24, SEQ ID NOS: 25 and 26, SEQ ID NOS: 27 and 28, and SEQ ID NOS: 29 and 30, as shown in Table 2. PCR was performed by 35 cycles consisting of denaturation at 95°C for 1 min; annealing at 55°C for 1 min; and polymerization reaction at 72°C for 1 min and 30 sec. The resulting DNA fragments were prepared as a single cassette through overlap extension PCR.

The cassette thus prepared was introduced into the strain prepared in Example 1-1 by a heat shock method, and then colonies were obtained, which were formed on a solid medium (YLMM1) without uracil. Colonies in which cassette insertion was confirmed using primers of SEQ ID NOS: 31 and 32 were plated on a 5-FOA solid medium and cultured at 30°C for 3 days, and colonies grown on the 5-FOA solid medium were obtained to recover the URA3 marker.

**[Table 2]**

| SEQ ID NO. | Sequence (5'-3') | PCR product |
|---|---|---|
| 21 | GACAATGCCTCGAGGAGGTTTAAAAGTAACT | Homology left arm |
| 22 | GCGCCGCCAACCCGGTCTCTCTGTGTTAGTCGGATGATAGG | |
| 23 | CCTATCATCCGACTAACACAGAGAGACCGGGTTGGCGGCGC | TEFINt promoter |
| 24 | GACGAGTCAGACAGGAGGCACTGCGGTTAGTACTGCAAAAAG | |
| 25 | CTTTTTGCAGTACTAACCGCAGTGCCTCCTGTCTGACTCGTC | URA3 |
| 26 | ATGCGCCGCCAACCCGGTCTCTTGGTGGTATTGTGACTGGGGAT | |
| 27 | ATCCCCAGTCACAATACCACCAAGAGACCGGGTTGGCGGCGCAT | Repeat region |
| 28 | CTTTCCAATAGCTGCTTGTAGCTGCGGTTAGTACTGCAAAA | |
| 29 | TTTTGCAGTACTAACCGCAGCTACAAGCAGCTATTGGAAAG | Homology right arm |
| 30 | GCTTAATGTGATTGATCTCAAACTTGATAG | |
| 31 | GCTGTCTCTGCGAGAGCACGTCGA | Forward |
| 32 | GGTTCGCACAACTTCTCGGGTGGC | Reverse |

### Example 1-3. Preparation of GGPPS-introduced strain

*Haematococcus pluvialis-derived* geranylgeranyl pyrophosphate synthase (GGPPS) gene was inserted into the genome of the strain which was prepared through Example 1-2.

A polynucleotide of SEQ ID NO: 33 of GGPPS was obtained, based on a nucleotide sequence (GenBank: APX64485.1) registered in the National Center for Biotechnology Information Search database (NCBI). Codon optimization of the polynucleotide sequence of GGPPS was performed to be suitable for *Y*. *lipolytica* through http://atgme.org, and the gene was synthesized by Macrogen in the form of TEFINtp-GGPPS-CYC1t (SEQ ID NO: 34). A cassette to be inserted into the LIG4(YALI0D21384g) gene site was designed using the URA3 gene (SEQ ID NO: 5) of Y. *lipolytica* as a selection marker.

Each PCR was performed using the synthesized GGPPS gene and genomic DNA of KCCM12972P as a template, and primers of SEQ ID NOS: 35 and 36, SEQ ID NOS: 37 and 38, SEQ ID NOS: 39 and 40, SEQ ID NOS: 41 and 42, and SEQ ID NOS: 43 and 44, as shown in Table 3. PCR was performed by 35 cycles consisting of denaturation at 95°C for 1 min; annealing at 55°C for 1 min; and polymerization reaction at 72°C for 2 min. The resulting DNA fragments were prepared as a single cassette through overlap extension PCR.

The cassette thus prepared was introduced into the strain prepared in Example 1-2 by a heat shock method, and then colonies were obtained, which were formed on a solid medium (YLMM1) without uracil. Colonies in which cassette insertion into the genome was confirmed using primers of SEQ ID NOS: 45 and 46 were plated on a 5-FOA solid medium and cultured at 30°C for 3 days, and colonies grown on the 5-FOA solid medium were obtained to recover the URA3 marker.

**[Table 3]**

| SEQ ID NO. | Sequence (5'-3') | PCR product |
|---|---|---|
| 35 | AAGACAAGGCTTCGGAAGCGAGAACCGCAA | Homology left arm |
| 36 | ATGCGCCGCCAACCCGGTCTCTGTGTTTGGCGGTGTGAGTTGTC | |
| 37 | GACAACTCACACCGCCAAACACAGAGACCGGGTTGGCGGCGCAT | Repeat region |
| 38 | ATGACGAGTCAGACAGGAGGCACTGCGGTTAGTACTGCAAAAAG | |
| 39 | CTTTTTGCAGTACTAACCGCAGTGCCTCCTGTCTGACTCGTCAT | URA3 |
| 40 | ATGCGCCGCCAACCCGGTCTCTTGGTGGTATTGTGACTGGGGAT | |
| 41 | ATCCCCAGTCACAATACCACCAAGAGACCGGGTTGGCGGCGCAT | TEFINtp-GGPPS-CYC1t |
| 42 | ATATGGAGTGTTATTTGAAGGGGCAAATTAAAGCCTTCGAGCGT | |
| 43 | ACGCTCGAAGGCTTTAATTTGCCCCTTCAAATAACACTCCATAT | Homology right arm |
| 44 | GTGTCCAAGTACGAACGCCAATGCAAGATT | |
| 45 | CCAGTTATTTGTACCATGCGGTGG | Forward |
| 46 | CCATCTTGTGTCGCGACGACGAAA | Reverse |

### Example 1-4. Preparation of KU80-deleted strain

To facilitate future strain preparation, KU80 (YALI0E02068g) gene was deleted. For this purpose, a KU80 gene deletion cassette was designed using the URA3 gene (SEQ ID NO: 5) of *Y*. *lipolytica* as a selection marker. Each PCR was performed using genomic DNA of KCCM12972P as a template, and primers of SEQ ID NOS: 47 and 48, SEQ ID NOS: 49 and 50, SEQ ID NOS: 51 and 52, and SEQ ID NOS: 53 and 54, as shown in Table 4. PCR was performed by 35 cycles consisting of denaturation at 95°C for 1 min; annealing at 55°C for 1 min; and polymerization reaction at 72°C for 1 min and 30 sec. The resulting DNA fragments were prepared as a single cassette through overlap extension PCR.

The cassette thus prepared was introduced into the strain prepared in Example 1-3 by a heat shock method, and then colonies were obtained, which were formed on a solid medium (YLMM1) without uracil. Colonies in which cassette insertion into the genome was confirmed using primers of SEQ ID NOS: 55 and 56 were plated on a 5-FOA solid medium and cultured at 30°C for 3 days, and colonies grown on the 5-FOA solid medium were obtained to recover the URA3 marker.

**[Table 4]**

| SEQ ID NO. | Sequence (5'-3') | PCR product |
|---|---|---|
| 47 | CCCACCTCCTCCTCCTGCTCCCCCGGCAGCCCCTGCCGCCCCTG | Homology left arm |
| 48 | ATGACGAGTCAGACAGGAGGCACCTAGTTAGTCAGAATTTTTGT | |
| 49 | ACAAAAATTCTGACTAACTAGGTGCCTCCTGTCTGACTCGTCAT | URA3 |
| 50 | TACCGGTCGGTAGCTACAATACTGGTGGTATTGTGACTGGGGAT | |
| 51 | ATCCCCAGTCACAATACCACCAGTATTGTAGCTACCGACCGGTA | Repeat region |
| 52 | CGTGTAGATCCACCACATACACCCTAGTTAGTCAGAATTTTTGT | |
| 53 | ACAAAAATTCTGACTAACTAGGGTGTATGTGGTGGATCTACACG | Homology right arm |
| 54 | AAGTAGGAAACATGATGGCCTCTTCTTCCTCTTTTGTAATGTAC | |
| 55 | CCCAACTCTCGAGGAAATGGCCAT | Forward |
| 56 | CTGGGGATCTTTTCCATCCTTGTT | Reverse |

### Example 1-5. Preparation of BLH-introduced strain

*Uncultured marine bacterium* 66A03-derived beta-carotene 15,15'-oxygenase (BLH) gene was inserted into the genome of the strain which was prepared through Example 1-4.

A polypeptide sequence of SEQ ID NO: 57 of BLH gene was obtained, based on an amino acid sequence (Q4PNI0) registered in the UniProtKB (UniProt Knowledgebase). Codon optimization thereof was performed to be suitable for Y. *lipolytica* through http://atgme.org, and the gene was synthesized by Macrogen in the form of TEFINtp-BLH-CYC1t (SEQ ID NO: 58). A cassette to be inserted into the KU70(YALI0C08701g) gene site was designed using the URA3 gene (SEQ ID NO: 5) of *Y*. *lipolytica* as a selection marker. Each PCR was performed using the synthesized BLH gene and genomic DNA of KCCM12972P as a template, and primers of SEQ ID NOS: 59 and 60, SEQ ID NOS: 61 and 62, SEQ ID NOS: 63 and 64, SEQ ID NOS: 65 and 66, and SEQ ID NOS: 67 and 68, as shown in Table 5. PCR was performed by 35 cycles consisting of denaturation at 95°C for 1 min; annealing at 55°C for 1 min; and polymerization reaction at 72°C for 2 min. The resulting DNA fragments were prepared as a single cassette through overlap extension PCR.

The cassette thus prepared was introduced into the strain prepared in Example 1-4 by a heat shock method, and then colonies were obtained, which were formed on a solid medium (YLMM1) without uracil. Colonies in which cassette insertion into the genome was confirmed using primers of SEQ ID NOS: 69 and 70 were plated on a 5-FOA solid medium and cultured at 30°C for 3 days, and colonies grown on the 5-FOA solid medium were obtained to recover the URA3 marker.

**[Table 5]**

| SEQ ID NO. | Sequence (5'-3') | PCR product |
|---|---|---|
| 59 | GTACCCGGGGATCCTCTAGAGGCGTTTCAGGTGGTTGCGTGAGTG | Homolog y left arm |
| 60 | GACACAAATGCGCCGCCAACCCGGTCTCTGCGGCGGTTCGTGGTTCGTG TTTC | |
| 61 | GAAACACGAACCACGAACCGCCGCAGAGACCGGGTTGGCGGCGCATTTG TGTC | TEFINtp-BLH-CYC1t |
| 62 | GACGAGTCAGACAGATACTCGTCGGCAAATTAAAGCCTTCGAGCGTCCC | |
| 63 | GGGACGCTCGAAGGCTTTAATTTGCCGACGAGTATCTGTCTGACTCGTC | URA3 |
| 64 | CAGGAAGAAGTAGATGCCGCCGCCGCAAAGGCCTGTTTCTCGGTGTACAG | |
| 65 | CTGTACACCGAGAAACAGGCCTTTGCGGCGGCGGCATCTACTTCTTCCTG | CYC1 terminate r |
| 66 | GCAGCAGTCATACATGTTCTGAGGCAAATTAAAGCCTTCGAGCGTCCC | |
| 67 | GGGACGCTCGAAGGCTTTAATTTGCCTCAGAACATGTATGACTGCTGC | Homolog y right arm |
| 68 | GCCTGCAGGTCGACTCTAGACTACTTTGTGCAGATTGAGGCCAAG | |
| 69 | CTTGACCTTGTAGAGCTGACCGGC | Forward |
| 70 | CACTACTTTCGCCACCAAGATGGG | Reverse |

### Example 2. Evaluation of impact of antioxidants on retinol production

A flask test was performed to compare the degree of retinol production of retinol-producing strains according to the presence and absence, and the type of antioxidants. Each retinol production platform strain was inoculated in a 250 ml corner-baffled flask containing 25 ml of *Yarrowia lipolytica* minimal media2 (YLMM2) at an initial OD of 4, and 4 types of antioxidants, 3,5-Di-tert-4-butylhydroxytoluene (BHT), propyl gallate (PG), vitamin C, and glutathione (GSH), were added to the medium at a concentration of 0.01% to 0.05%, respectively. Culturing was carried out under conditions of 30°C and 200 rpm.

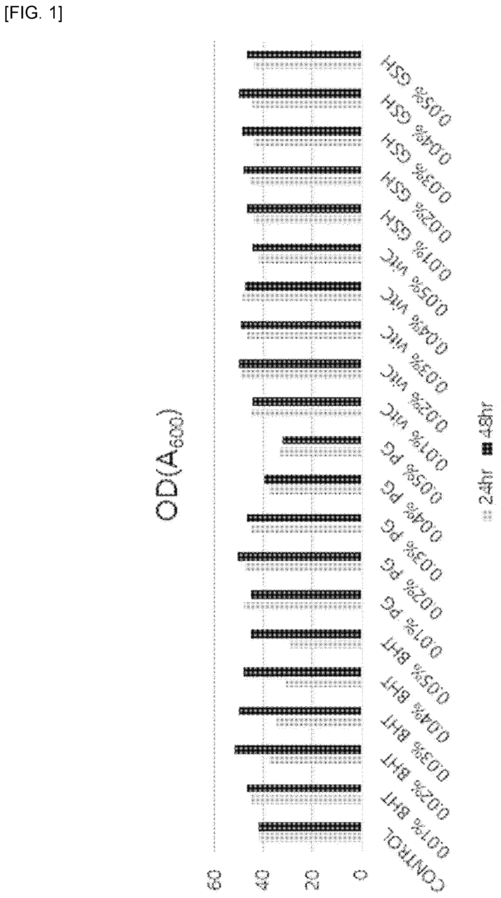

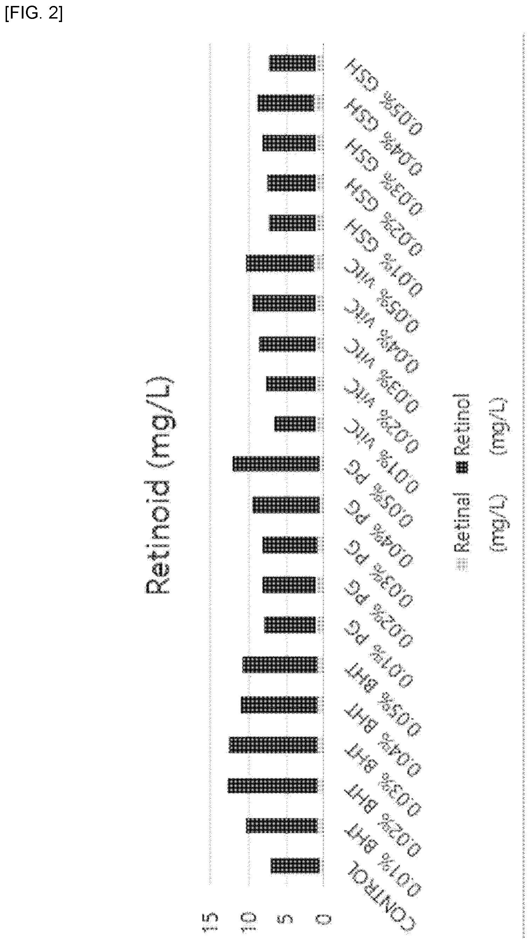

To evaluate the degree of growth according to culture time, the OD value at a wavelength of 600 nm was measured using a spectrophotometer, and the method of measuring retinol concentrations is as follows; After the culture was completed, 1 ml of the culture medium was centrifuged to remove the supernatant, then 0.5 ml of dimethyl sulfoxide (DMSO, Sigma) was added and the cells were disrupted by agitation at 55 °C for 10 minutes (agitation; 2,000 rpm). Next, 0.5 ml of acetone (Sigma) containing 4% BHT was added and agitated (2,000 rpm) for 15 min at 45 °C, and retinoids extracted in this manner were quantitatively analyzed using HPLC equipment. The analyzed OD values are shown in FIG. 1 and the retinoid concentrations are shown in FIG. 2.

Considering that the sugar consumption rate may vary depending on the type of antioxidants which were added to the medium, BHT, PG, vitamin C, and GSH were each cultured for 48 hours. After 48 hour-incubation, retinal and retinol were extracted and quantified.

As a result, when BHT, PG, vitamin C, and GSH were added, the retinol concentration increased by up to 1.89 times, 1.82 times, 1.44 times, and 1.21 times, respectively, as compared to the control group without antioxidants, and in some cases, biomass (OD) was overall high, as compared to that without addition, indicating that antioxidants also promote the growth of microorganisms (FIG. 2).

The above results confirmed that when the retinol-producing strains are cultured, addition of antioxidants to the medium not only promotes the growth of the retinol-producing strains, but also increases the retinol production concentration.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

Each sequence according to SEQ ID NO. of the present disclosure is shown in Table 6 below.

**[Table 6]**

| SEQ ID NO. | Name | Sequence | Type |
|---|---|---|---|
| 1 | crtYB | atgacggctc tcgcatatta ccagatccat ctgatctata ctctcccaat tcttggtctt 60 ctcggcctgc tcacttcccc gattttgaca aaatttgaca tctacaaaat atcgatcctc 120 gtatttattg cgtttagtgc aaccacacca tgggactcat ggatcatcag aaatggcgca 180 tggacatatc catcagcgga gagtggccaa ggcgtgtttg gaacgtttct agatgttcca 240 tatgaagagt acgctttctt tgtcattcaa accgtaatca ccggcttggt ctacgtcttg 300 gcaactaggc accttctccc atctctcgcg cttcccaaga ctagatcgtc cgccctttct 360 ctcgcgctca aggcgctcat ccctctgccc attatctacc tatttaccgc tcaccccagc 420 ccatcgcccg acccgctcgt gacagatcac tacttctaca tgcgggcact ctccttactc 480 atcaccccac ctaccatgct cttggcagca ttatcaggcg aatatgcttt cgattggaaa 540 agtggccgag caaagtcaac tattgcagca atcatgatcc cgacggtgta tctgatttgg 600 gtagattatg ttgctgtcgg tcaagactct tggtcgatca acgatgagaa gattgtaggg 660 | DNA |
| | | tggaggcttg gaggtgtact acccattgag gaagctatgt tcttcttact gacgaatcta 720 atgattgttc tgggtctgtc tgcctgcgat catactcagg ccctatacct gctacacggt 780 cgaactattt atggcaacaa aaagatgcca tcttcatttc ccctcattac accgcctgtg 840 ctctccctgt tttttagcag ccgaccatac tcttctcagc caaaacgtga cttggaactg 900 gcagtcaagt tgttggagga aaagagccgg agcttttttg ttgcctcggc tggatttcct 960 agcgaagtta gggagaggct ggttggacta tacgcattct gccgggtgac tgatgatctt 1020 atcgactctc ctgaagtatc ttccaacccg catgccacaa ttgacatggt ctccgatttt 1080 | |
| | | cttaccctac tatttgggcc cccgctacac ccttcgcaac ctgacaagat cctttcttcg 1140 cctttacttc ctccttcgca cccttcccga cccacgggaa tgtatcccct cccgcctcct 1200 ccttcgctct cgcctgccga gctcgttcaa ttccttaccg aaagggttcc cgttcaatac 1260 catttcgcct tcaggttgct cgctaagttg caagggctga tccctcgata cccactcgac 1320 gaactcctta gaggatacac cactgatctt atctttccct tatcgacaga ggcagtccag 1380 gctcggaaga cgcctatcga gaccacagct gacttgctgg actatggtct atgtgtagca 1440 ggctcagtcg ccgagctatt ggtctatgtc tcttgggcaa gtgcaccaag tcaggtccct 1500 gccaccatag aagaaagaga agctgtgtta gtggcaagcc gagagatggg aactgccctt 1560 cagttggtga acattgctag ggacattaaa ggggacgcaa cagaagggag attttaccta 1620 ccactctcat tctttggtct tcgggatgaa tcaaagcttg cgatcccgac tgattggacg 1680 gaacctcggc ctcaagattt cgacaaactc ctcagtctat ctccttcgtc cacattacca 1740 | |
| | | tcttcaaacg cctcagaaag cttccggttc gaatggaaga cgtactcgct tccattagtc 1800 gcctacgcag aggatcttgc caaacattct tataagggaa ttgaccgact tcctaccgag 1860 gttcaagcgg gaatgcgagc ggcttgcgcg agctacctac tgatcggccg agagatcaaa 1920 gtcgtttgga aaggagacgt cggagagaga aggacagttg ccggatggag gagagtacgg 1980 aaagtcttga gtgtggtcat gagcggatgg gaagggcagt aa | |
| 2 | crtl | atgggaaaag aacaagatca ggataaaccc acagctatca tcgtgggatg tggtatcggt 60 ggaatcgcca ctgccgctcg tcttgctaaa gaaggtttcc aggtcacggt gttcgagaag 120 aacgactact ccggaggtcg atgctcttta atcgagcgag atggttatcg attcgatcag 180 gggcccagtt tgctgctctt gccagatctc ttcaagcaga cattcgaaga tttgggagag 240 aagatggaag attgggtcga tctcatcaag tgtgaaccca actatgtttg ccacttccac 300 gatgaagaga ctttcactct ttcaaccgac atggcgttgc tcaagcggga agtcgagcgt 360 tttgaaggca aagatggatt tgatcggttc ttgtcgttta tccaagaagc ccacagacat 420 tacgagcttg ctgtcgttca cgtcctgcag aagaacttcc ctggcttcgc agcattctta 480 cggctacagt tcattggcca aatcctggct cttcacccct tcgagtctat ctggacaaga 540 gtttgtcgat atttcaagac cgacagatta cgaagagtct tctcgtttgc agtgatgtac 600 atgggtcaaa gcccatacag tgcgcccgga acatattcct tgctccaata caccgaattg 660 accgagggca tctggtatcc gagaggaggc ttttggcagg ttcctaatac tcttcttcag 720 atcgtcaagc gcaacaatcc ctcagccaag ttcaatttca acgctccagt ttcccaggtt 780 cttctctctc ctgccaagga ccgagcgact ggtgttcgac ttgaatccgg cgaggaacat 840 | DNA |
| | | cacgccgatg ttgtgattgt caatgctgac ctcgtttacg cctccgagca cttgattcct 900 gacgatgcca gaaacaagat tggccaactg ggtgaagtca agagaagttg gtgggctgac 960 ttagttggtg gaaagaagct caagggaagt tgcagtagtt tgagcttcta ctggagcatg 1020 gaccgaatcg tggacggtct gggcggacac aatatcttct tggccgagga cttcaaggga 1080 | |
| | | tcattcgaca caatcttcga ggagttgggt ctcccagccg atccttcctt ttacgtgaac 1140 gttccctcgc gaatcgatcc ttctgccgct cccgaaggca aagatgctat cgtcattctt 1200 gtgccgtgtg gccatatcga cgcttcgaac cctcaagatt acaacaagct tgttgctcgg 1260 gcaaggaagt ttgtgatcca cacgctttcc gccaagcttg gacttcccga ctttgaaaaa 1320 atgattgtgg cagagaaggt tcacgatgct ccctcttggg agaaagaatt caacctcaag 1380 gacggaagca tcttgggact ggctcacaac tttatgcaag ttcttggttt caggccgagc 1440 accagacatc ccaagtatga caagttgttc tttgtcgggg cttcgactca tcccggaact 1500 ggggttccca tcgtcttggc tggagccaag ttaactgcca accaagttct cgaatccttt 1560 gaccgatccc cagctccaga tcccaatatg tcactctccg taccatatgg aaaacctctc 1620 aaatcaaatg gaacgggtat cgattctcag gtccagctga agttcatgga tttggagaga 1680 tgggtatacc ttttggtgtt gttgattggg gccgtgatcg ctcgatccgt tggtgttctt 1740 gctttctga | |
| 3 | TEFINtp-crtYB-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt | DNA |
| | | ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gacggctctc 540 gcatattacc agatccatct gatctatact ctcccaattc ttggtcttct cggtctgctc 600 acttccccga ttttgacaaa atttgacatc tacaaaatat cgatcctcgt atttattgcg 660 tttagtgcaa ccacaccatg ggactcatgg atcatcagaa atggcgcatg gacatatcca 720 tcagcggaga gtggccaagg cgtgtttgga acgtttctag atgttccata tgaagagtac 780 gctttctttg tcattcaaac cgtaatcacc ggcttggtct acgtcttggc aactaggcac 840 cttctcccat ctctcgcgct tcccaagact agatcgtccg ccctttctct cgcgctcaag 900 gcgctcatcc ctctgcccat tatctaccta tttaccgctc accccagccc atcgcccgac 960 ccgctcgtga cagatcacta cttctacatg cgggcactct ccttactcat caccccacct 1020 accatgctct tggcagcatt atcaggcgaa tatgctttcg attggaaaag tggccgagca 1080 | |
| | | aagtcaacta ttgcagcaat catgatcccg acggtgtatc tgatttgggt agattatgtt 1140 gctgtcggtc aagactcttg gtcgatcaac gatgagaaga ttgtagggtg gaggcttgga 1200 ggtgtactac ccattgagga agctatgttc ttcttactga cgaatctaat gattgttctg 1260 | |
| | | ggtctgtctg cctgcgatca tactcaggcc ctatacctgc tacacggtcg aactatttat 1320 ggcaacaaaa agatgccatc ttcatttccc ctcattacac cgcctgtgct ctccctgttt 1380 tttagcagcc gaccatactc ttctcagcca aaacgtgact tggaactggc agtcaagttg 1440 ttggaggaaa agagccggag cttttttgtt gcctcggctg gatttcctag cgaagttagg 1500 gagaggctgg ttggactata cgcattctgc cgggtgactg atgatcttat cgactctcct 1560 gaagtatctt ccaacccgca tgccacaatt gacatggtct ccgattttct taccctacta 1620 tttgggcccc cgctacaccc ttcgcaacct gacaagatcc tttcttcgcc tttacttcct 1680 ccttcgcacc cttcccgacc cacgggaatg tatcccctcc cgcctcctcc ttcgctctcg 1740 cctgccgagc tcgttcaatt ccttaccgaa agggttcccg ttcaatacca tttcgccttc 1800 aggttgctcg ctaagttgca agggctgatc cctcgatacc cactcgacga actccttaga 1860 ggatacacca ctgatcttat ctttccttta tcgacagagg cagtccaggc tcggaagacg 1920 cctatcgaga ccacagctga cttgctggac tatggtctat gtgtagcagg ctcagtcgcc 1980 gagctattgg tctatgtctc ttgggcaagt gcaccaagtc aggtccctgc caccatagaa 2040 gaaagagaag ctgtgttagt ggcaagccga gagatgggaa ctgcccttca gttggtgaac 2100 attgctaggg acattaaagg ggacgcaaca gaagggagat tttacctacc actctcattc 2160 | |
| | | tttggtcttc gggatgaatc aaagcttgcg atcccgactg attggacgga acctcggcct 2220 caagatttcg acaaactcct cagtctatct ccttcgtcca cattaccatc ttcaaacgcc 2280 tcagaaagct tccggttcga atggaagacg tactcgcttc cattagtcgc ctacgcagag 2340 gatcttgcca aacattctta taagggaatt gaccgacttc ctaccgaggt | |
| | | tcaagcggga 2400 atgcgagcgg cttgcgcgag ctacctactg atcggccgag agatcaaagt cgtttggaaa 2460 ggagacgtcg gagagagaag gacagttgcc ggatggagga gagtacggaa agtcttgagt 2520 gtggtcatga gcggatggga agggcagtaa ctcgagtcat gtaattagtt atgtcacgct 2580 tacattcacg ccctcccccc acatccgctc taaccgaaaa ggaaggagtt agacaacctg 2640 aagtctaggt ccctatttat ttttttatag ttatgttagt attaagaacg ttatttatat 2700 ttcaaatttt tctttttttt ctgtacagac gcgtgtacgc atgtaacatt atactgaaaa 2760 ccttgcttga gaaggttttg ggacgctcga aggctttaat ttgc | |
| 4 | TEFINtp-crtl-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gggaaaagaa 540 caagatcagg ataaacccac agctatcatc gtgggatgtg gtatcggtgg aatcgccact 600 gccgctcgtc ttgctaaaga aggtttccag gtcacggtgt tcgagaagaa cgactactcc 660 ggaggtcgat gctctttaat cgagcgagat ggttatcgat tcgatcaggg | DNA |
| | | gcccagtttg 720 ctgctcttgc cagatctctt caagcagaca ttcgaagatt tgggagagaa gatggaagat 780 tgggtcgatc tcatcaagtg tgaacccaac tatgtttgcc acttccacga tgaagagact 840 ttcactcttt caaccgacat ggcgttgctc aagcgggaag tcgagcgttt tgaaggcaaa 900 gatggatttg atcggttctt gtcgtttatc caagaagccc acagacatta cgagcttgct 960 gtcgttcacg tcctgcagaa gaacttccct ggcttcgcag cattcttacg gctacagttc 1020 attggccaaa tcctggctct tcaccccttc gagtctatct ggacaagagt ttgtcgatat 1080 | |
| | | ttcaagaccg acagattacg aagagtcttc tcgtttgcag tgatgtacat gggtcaaagc 1140 ccatacagtg cgcccggaac atattccttg ctccaataca ccgaattgac cgagggcatc 1200 tggtatccga gaggaggctt ttggcaggtt cctaatactc ttcttcagat cgtcaagcgc 1260 aacaatccct cagccaagtt caatttcaac gctccagttt cccaggttct tctctctcct 1320 gccaaggacc gagcgactgg tgttcgactt gaatccggcg aggaacatca cgccgatgtt 1380 gtgattgtca atgctgacct cgtttacgcc tccgagcact tgattcctga cgatgccaga 1440 aacaagattg gccaactggg tgaagtcaag agaagttggt gggctgactt agttggtgga 1500 aagaagctca agggaagttg cagtagtttg agcttctact ggagcatgga ccgaatcgtg 1560 gacggtctgg gcggacacaa tatcttcttg gccgaggact tcaagggatc attcgacaca 1620 atcttcgagg agttgggtct cccagccgat ccttcctttt acgtgaacgt tccctcgcga 1680 atcgatcctt ctgccgctcc cgaaggcaaa gatgctatcg tcattcttgt gccgtgtggc 1740 catatcgacg cttcgaaccc tcaagattac aacaagcttg ttgctcgggc aaggaagttt 1800 | |
| | | gtgatccaca cgctttccgc caagcttgga cttcccgact ttgaaaaaat gattgtggca 1860 gagaaggttc acgatgctcc ctcttgggag aaagaattca acctcaagga cggaagcatc 1920 ttgggactgg ctcacaactt tatgcaagtt cttggtttca ggccgagcac cagacatccc 1980 aagtatgaca agttgttctt tgtcggggct tcgactcatc ccggaactgg ggttcccatc 2040 | |
| | | gtcttggctg gagccaagtt aactgccaac caagttctcg aatcctttga ccgatcccca 2100 gctccagatc ccaatatgtc actctccgta ccatatggaa aacctctcaa atcaaatgga 2160 acgggtatcg attctcaggt ccagctgaag ttcatggatt tggagagatg ggtatacctt 2220 ttggtattgt tgattggggc cgtgatcgct cgatccgttg gtgttcttgc tttctgactc 2280 gagtcatgta attagttatg tcacgcttac attcacgccc tccccccaca tccgctctaa 2340 ccgaaaagga aggagttaga caacctgaag tctaggtccc tatttatttt tttatagtta 2400 tgttagtatt aagaacgtta tttatatttc aaatttttct tttttttctg tacagacgcg 2460 tgtacgcatg taacattata ctgaaaacct tgcttgagaa ggttttggga cgctcgaagg 2520 ctttaatttg c | |
| 5 | URA3 | tgcctcctgt ctgactcgtc attgccgcct ttggagtacg actccaacta tgagtgtgct 60 tggatcactt tgacgataca ttcttcgttg gaggctgtgg gtctgacagc tgcgttttcg 120 gcgcggttgg ccgacaacaa tatcagctgc aacgtcattg ctggctttca tcatgatcac 180 atttttgtcg gcaaaggcga cgcccagaga gccattgacg ttctttctaa tttggaccga 240 tagccgtata gtccagtcta tctataagtt caactaactc gtaactatta ccataacata 300 tacttcactg ccccagataa ggttccgata aaaagttctg cagactaaat | DNA |
| | | ttatttcagt 360 ctcctcttca ccaccaaaat gccctcctac gaagctcgag ctaacgtcca caagtccgcc 420 tttgccgctc gagtgctcaa gctcgtggca gccaagaaaa ccaacctgtg tgcttctctg 480 gatgttacca ccaccaagga gctcattgag cttgccgata aggtcggacc ttatgtgtgc 540 atgatcaaga cccatatcga catcattgac gacttcacct acgccggcac tgtgctcccc 600 ctcaaggaac ttgctcttaa gcacggtttc ttcctgttcg aggacagaaa gttcgcagat 660 attggcaaca ctgtcaagca ccagtacaag aacggtgtct accgaatcgc cgagtggtcc 720 gatatcacca acgcccacgg tgtacccgga accggaatca ttgctggcct gcgagctggt 780 gccgaggaaa ctgtctctga acagaagaag gaggacgtct ctgactacga gaactcccag 840 tacaaggagt tcctggtccc ctctcccaac gagaagctgg ccagaggtct gctcatgctg 900 gccgagctgt cttgcaaggg ctctctggcc actggcgagt actccaagca gaccattgag 960 cttgcccgat ccgaccccga gtttgtggtt ggcttcattg cccagaaccg acctaagggc 1020 | |
| | | gactctgagg actggcttat tctgaccccc ggggtgggtc ttgacgacaa gggagacgct 1080 ctcggacagc agtaccgaac tgttgaggat gtcatgtcta ccggaacgga tatcataatt 1140 gtcggccgag gtctgtacgg ccagaaccga gatcctattg aggaggccaa gcgataccag 1200 aaggctggct gggaggctta ccagaagatt aactgttaga ggttagacta tggatatgtc 1260 atttaactgt gtatatagag agcgtgcaag tatggagcgc ttgttcagct tgtatgatgg 1320 tcagacgacc tgtctgatcg agtatgtatg atactgcaca acctgtgtat ccgcatgatc 1380 tgtccaatgg ggcatgttgt tgtgtttctc gatacggaga tgctgggtac aagtagctaa 1440 | |
| | | tacgattgaa ctacttatac ttatatgagg cttgaagaaa gctgacttgt gtatgactta 1500 ttctcaacta catccccagt cacaatacca cca | |
| 6 | primer | gtgcgcttct ctcgtctcgg taaccctgtc | DNA |
| 7 | primer | atgcgccgcc aacccggtct ctggggtgtg gtggatgggg tgtg | DNA |
| 8 | primer | cacaccccat ccaccacacc ccagagaccg ggttggcggc gcat | DNA |
| 9 | primer | cgccgccaac ccggtctctt gaagacgaaa gggcctccg | DNA |
| 10 | primer | cggaggccct ttcgtcttca agagaccggg ttggcggcg | DNA |
| 11 | primer | gacgagtcag acaggaggca tcagacagat actcgtcgcg | DNA |
| 12 | primer | cgcgacgagt atctgtctga tgcctcctgt ctgactcgtc | DNA |
| 13 | primer | atgacgagtc agacaggagg catggtggta ttgtgactgg ggat | DNA |
| 14 | primer | atccccagtc acaataccac catgcctcct gtctgactcg teat | DNA |
| 15 | primer | cggcgtcctt ctcgtagtcc gcttttggtg gtgaagagga gact | DNA |
| 16 | primer | agtctcctct tcaccaccaa aagcggacta cgagaaggac gccg | DNA |
| 17 | primer | ccactcgtca ccaacagtgc cgtgtgttgc | DNA |
| 18 | primer | tcgtacgtct ataccaacag atgg | DNA |
| 19 | primer | cgcatacaca cacactgccg gggg | DNA |
| 20 | HMGR native promoter | tccacacgtc gttctttttt ccttagcctt ttttgcagtg cgcgtgtccc aaaccccagc 60 tctacacacc agcacaaaca aagttaagct cagggttgtc gttgaggtcg cttactgtag 120 tcagtgctcg tatggttcgt tcaattttcg ccaaaaatcg ttttgccttt gtatcttggg 180 aataacatca actgtggttc ttcaacaggc ctaaggaacg aaacaagccg gaccaagatc 240 aggttcaagg tgagtactga gaaggaatag aaggcctaaa ggcgcaaacc gacaggtggc 300 aacagctcca caccgaccac gaaggccacg aaatcaaggg gtcctaaagt tagtctttgt 360 ggcctcgacg gtcagcgaaa acgcgagacc acaacgcgat cagaaccagg acctaaacaa 420 cacaggacgg ggtcacaata ggcttgaaca gcaagtacaa gctgtgatct ctctatattt 480 gattctcaaa ccacccctga ctacttcagc gcctctgtga cacagccccc ctatcatccg 540 actaacacag | DNA |
| 21 | Primer | gacaatgcct cgaggaggtt taaaagtaac t | DNA |
| 22 | Primer | gcgccgccaa cccggtctct ctgtgttagt cggatgatag g | DNA |
| 23 | Primer | cctatcatcc gactaacaca gagagaccgg gttggcggcg c | DNA |
| 24 | Primer | gacgagtcag acaggaggca ctgcggttag tactgcaaaa ag | DNA |
| 25 | Primer | ctttttgcag tactaaccgc agtgcctcct gtctgactcg tc | DNA |
| 26 | Primer | atgcgccgcc aacccggtct cttggtggta ttgtgactgg ggat | DNA |
| 27 | Primer | atccccagtc acaataccac caagagaccg ggttggcggc gcat | DNA |
| 28 | Primer | ctttccaata gctgcttgta gctgcggtta gtactgcaaa a | DNA |
| 29 | Primer | ttttgcagta ctaaccgcag ctacaagcag ctattggaaa g | DNA |
| 30 | Primer | gcttaatgtg attgatctca aacttgatag | DNA |
| 31 | Primer | gctgtctctg cgagagcacg tcga | DNA |
| 32 | Primer | ggttcgcaca acttctcggg tggc | DNA |
| 33 | ggpps | atgatccgag cgatgcacaa ccgggcgccc acacctcgaa ctcgagtgtc tcatccacgc 60 tcacataggg ctctggcaca tgtctcagcc gtagcaacag cagggcaggt ggcagaggtc 120 cactctgctc ctgcctttga cttcgagatg tacatgagag acagagctga gatggtcaac 180 aaggctcttg atgctgcatt gccatctaga taccctgagg tgctggttga ttccatgagg 240 tactccgtac ttgcgggtgg caagcgcgtg aggcctgccc tgacactggc tgcgtgcgac 300 cttgtaggag gggacatggc cactgcccta cccaccgcat gtgccatgga gatgatccac 360 accatgagcc tcatccatga tgacctgcca gccatggaca atgacgactt caggcgaggt 420 cggcccacaa accataaggt gtatggtgag gacattgcca tccttgctgg tgacgcgctg 480 ctgtcctttg cctttgagca catcgcccgg gacaccaaag gcgtgccggc tgatgcggtg 540 ctgaaggtca tcatggagct gggccgggcc gtgggcgcgc agggcctatc agcaggccag 600 gctgttgaca taaagagcga gggccaggag gtggggctgg aggtgctgga gtacatccac 660 caccacaaga cagctgcact gctggaggcg gcagtggtgt gcggcgcact ggtgggcggg 720 | DNA |
| | | gccgacaccg ccaccgtgga gaagctgcgc aagtacgcgc tgaacattgg gctggccttc 780 caggtgattg atgacatcct ggacgtcact caaaccaccg aaaccttggg caagaccgca 840 gccaaagacc tggcggtgaa caagaccacc tatcccaagc tgctgggtct ggaagccagc 900 aggaaggtgg cggacgactt gatcagggag gctatagcac agttagacga gtttgagcct 960 gcacgcaagg cgcccatggt ggccctggcc cacctcatag ggtaccgcaa gaactga | |
| 34 | TEFINtp-GGPPS-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gatccgagcc 540 atgcacaacc gagcccccac cccccgaacc cgagtgtctc acccccgatc tcaccgagcc 600 ctggcccacg tgtctgccgt ggccaccgcc ggccaggtgg ccgaggtgca ctctgccccc 660 gccttcgact tcgagatgta catgcgagac cgagccgaga tggtgaacaa ggccctggac 720 gccgccctgc cctctcgata ccccgaggtg ctggtggact ctatgcgata ctctgtgctg 780 gccggcggca agcgagtgcg acccgccctg accctggccg cctgtgacct | DNA |
| | | ggtgggcggc 840 gacatggcca ccgccctgcc caccgcctgt gccatggaga tgatccacac catgtctctg 900 atccacgacg acctgcccgc catggacaac gacgacttcc gacgaggccg acccaccaac 960 cacaaggtgt acggcgagga catcgccatc ctggccggcg acgccctgct gtctttcgcc 1020 ttcgagcaca tcgcccgaga caccaagggc gtgcccgccg acgccgtgct gaaggtgatc 1080 | |
| | | atggagctgg gccgagccgt gggcgcccag ggcctgtctg ccggccaggc cgtggacatc 1140 aagtctgagg gccaggaggt gggcctggag gtgctggagt acatccacca ccacaagacc 1200 gccgccctgc tggaggccgc cgtggtgtgt ggcgccctgg tgggcggcgc cgacaccgcc 1260 accgtggaga agctgcgaaa gtacgccctg aacatcggcc tggccttcca ggtgatcgac 1320 gacatcctgg acgtgaccca gaccaccgag accctgggca agaccgccgc caaggacctg 1380 gccgtgaaca agaccaccta ccccaagctg ctgggcctgg aggcctctcg aaaggtggcc 1440 gacgacctga tccgagaggc catcgcccag ctggacgagt tcgagcccgc ccgaaaggcc 1500 cccatggtgg ccctggccca cctgatcggc taccgaaaga actagtcatg taattagtta 1560 tgtcacgctt acattcacgc cctccctcca catccgctct aaccgaaaag gaaggagtta 1620 gacaacctga agtctaggtc cctatttatt tttttatagt tatgttagta ttaagaacgt 1680 tatttatatt tcaaattttt cttttttttc tgtacagacg cgtgtacgca tgtaacatta 1740 tactgaaaac cttgcttgag aaggttttgg gacgctcgaa ggctttaatt tgc | |
| 35 | primer | aagacaaggc ttcggaagcg agaaccgcaa | DNA |
| 36 | primer | atgcgccgcc aacccggtct ctgtgtttgg cggtgtgagt tgtc | DNA |
| 37 | primer | gacaactcac accgccaaac acagagaccg ggttggcggc gcat | DNA |
| 38 | primer | atgacgagtc agacaggagg cactgcggtt agtactgcaa aaag | DNA |
| 39 | primer | ctttttgcag tactaaccgc agtgcctcct gtctgactcg teat | DNA |
| 40 | primer | atgcgccgcc aacccggtct cttggtggta ttgtgactgg ggat | DNA |
| 41 | primer | atccccagtc acaataccac caagagaccg ggttggcggc gcat | DNA |
| 42 | primer | atatggagtg ttatttgaag gggcaaatta aagccttcga gcgt | DNA |
| 43 | primer | acgctcgaag gctttaattt gccccttcaa ataacactcc atat | DNA |
| 44 | primer | gtgtccaagt acgaacgcca atgcaagatt | DNA |
| 45 | primer | ccagttattt gtaccatgcg gtgg | DNA |
| 46 | primer | ccatcttgtg tcgcgacgac gaaa | DNA |
| 47 | primer | cccacctcct cctcctgctc ccccggcagc ccctgccgcc cctg | DNA |
| 48 | primer | atgacgagtc agacaggagg cacctagtta gtcagaattt ttgt | DNA |
| 49 | primer | acaaaaattc tgactaacta ggtgcctcct gtctgactcg teat | DNA |
| 50 | primer | taccggtcgg tagctacaat actggtggta ttgtgactgg ggat | DNA |
| 51 | primer | atccccagtc acaataccac cagtattgta gctaccgacc ggta | DNA |
| 52 | primer | cgtgtagatc caccacatac accctagtta gtcagaattt ttgt | DNA |
| 53 | primer | acaaaaattc tgactaacta gggtgtatgt ggtggatcta cacg | DNA |
| 54 | primer | aagtaggaaa catgatggcc tcttcttcct cttttgtaat gtac | DNA |
| 55 | primer | cccaactctc gaggaaatgg ccat | DNA |
| 56 | primer | ctggggatct tttccatcct tgtt | DNA |
| 57 | BLH | | Protein |
| 58 | TEFINtp-BLH-CYC1t | agagaccggg ttggcggcgc atttgtgtcc caaaaaacag ccccaattgc cccaattgac 60 cccaaattga cccagtagcg ggcccaaccc cggcgagagc ccccttctcc ccacatatca 120 aacctccccc ggttcccaca cttgccgtta agggcgtagg gtactgcagt ctggaatcta 180 cgcttgttca gactttgtac tagtttcttt gtctggccat ccgggtaacc catgccggac 240 gcaaaataga ctactgaaaa tttttttgct ttgtggttgg gactttagcc aagggtataa 300 | DNA |
| | | aagaccaccg tccccgaatt acctttcctc ttcttttctc tctctccttg tcaactcaca 360 cccgaaatcg ttaagcattt ccttctgagt ataagaatca ttcaaaatgg tgagtttcag 420 aggcagcagc aattgccacg ggctttgagc acacggccgg gtgtggtccc attcccatcg 480 acacaagacg ccacgtcatc cgaccagcac tttttgcagt actaaccgca gggcctgatg 540 ctgatcgact ggtgtgccct ggccctggtg gtgttcatcg gcctgcccca cggcgccctg 600 gacgccgcca tctctttctc tatgatctct tctgccaagc gaatcgcccg actggccggc 660 atcctgctga tctacctgct gctggccacc gccttcttcc tgatctggta ccagctgccc 720 gccttctctc tgctgatctt cctgctgatc tctatcatcc acttcggcat ggccgacttc 780 aacgcctctc cctctaagct gaagtggccc cacatcatcg cccacggcgg cgtggtgacc 840 gtgtggctgc ccctgatcca gaagaacgag gtgaccaagc tgttctctat cctgaccaac 900 ggccccaccc ccatcctgtg ggacatcctg ctgatcttct tcctgtgttg gtctatcggc 960 gtgtgtctgc acacctacga gaccctgcga tctaagcact acaacatcgc cttcgagctg 1020 atcggcctga tcttcctggc ctggtacgcc ccccccctgg | |
| | | tgaccttcgc cacctacttc 1080 tgtttcatcc actctcgacg acacttctct ttcgtgtgga agcagctgca gcacatgtct 1140 tctaagaaga tgatgatcgg ctctgccatc atcctgtctt gtacctcttg gctgatcggc 1200 ggcggcatct acttcttcct gaactctaag atgatcgcct ctgaggccgc cctgcagacc 1260 gtgttcatcg gcctggccgc cctgaccgtg ccccacatga tcctgatcga cttcatcttc 1320 cgaccccact cttctcgaat caagatcaag aactagtcat gtaattagtt atgtcacgct 1380 tacattcacg ccctccctcc acatccgctc taaccgaaaa ggaaggagtt | |
| | | agacaacctg 1440 aagtctaggt ccctatttat ttttttatag ttatgttagt attaagaacg ttatttatat 1500 ttcaaatttt tctttttttt ctgtacagac gcgtgtacgc atgtaacatt atactgaaaa 1560 ccttgcttga gaaggttttg ggacgctcga aggctttaat ttgc | |
| 59 | primer | gtacccgggg atcctctaga ggcgtttcag gtggttgcgt gagtg | DNA |
| 60 | primer | gacacaaatg cgccgccaac ccggtctctg cggcggttcg tggttcgtgt ttc | DNA |
| 61 | primer | gaaacacgaa ccacgaaccg ccgcagagac cgggttggcg gcgcatttgt gtc | DNA |
| 62 | primer | gacgagtcag acagatactc gtcggcaaat taaagccttc gagcgtccc | DNA |
| 63 | primer | gggacgctcg aaggctttaa tttgccgacg agtatctgtc tgactcgtc | DNA |
| 64 | primer | caggaagaag tagatgccgc cgccgcaaag gcctgtttct cggtgtacag | DNA |
| 65 | primer | ctgtacaccg agaaacaggc ctttgcggcg gcggcatcta cttcttcctg | DNA |
| 66 | primer | gcagcagtca tacatgttct gaggcaaatt aaagccttcg agcgtccc | DNA |
| 67 | primer | gggacgctcg aaggctttaa tttgcctcag aacatgtatg actgctgc | DNA |
| 68 | primer | gcctgcaggt cgactctaga ctactttgtg cagattgagg ccaag | DNA |
| 69 | primer | cttgaccttg tagagctgac cggc | DNA |
| 70 | primer | cactactttc gccaccaaga tggg | DNA |

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Yarrowia lipolytica CC08-0125 | Accession number given by the
INTERNATIONAL DEPOSITARY AUTHORITY:
KCCM12972P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on April. 09. 2021 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: April. 09. 2021. 한국미생물보존센터장인
¹ Where Rule 6.4(d) applies, such date is the date on which the status of international depositary authority was
acquired.

Form BP/4 (sole page)

## Claims

1. A method of producing retinol, the method comprising the step of culturing a microorganism of the genus *Yarrowia* in a medium comprising an antioxidant.

2. The method of claim 1, wherein the antioxidant is any one or more selected from the group consisting of 3,5-di-tert-4-butylhydroxytoluene (BHT), propyl gallate (PG), vitamin C (ascorbic acid), and glutathione (GSH).

3. The method of claim 1, wherein the microorganism is *Yarrowia lipolytica.*

4. The method of claim 1, wherein the microorganism is for producing retinol.

5. The method of claim 1, wherein the antioxidant is included at a concentration of 0.001%(w/v) or more with respect to the total medium composition.

6. The method of claim 1, further comprising the step of recovering retinol from the medium or microorganism.

7. A method of increasing retinol production, the method comprising the step of culturing a microorganism of the genus *Yarrowia* in a medium comprising an antioxidant.

8. A method of producing retinoids, the method comprising the steps of:
culturing a microorganism of the genus *Yarrowia* in a medium comprising an antioxidant; and
converting retinol, which is produced by the microorganism, into retinoids other than retinol.

9. A medium composition for a microorganism of the genus *Yarrowia* for producing retinol, the composition comprising an antioxidant.

10. The medium composition of claim 9, wherein the antioxidant is any one or more selected from the group consisting of 3,5-di-tert-4-butylhydroxytoluene (BHT), propyl gallate (PG), vitamin C (ascorbic acid), and glutathione (GSH).

11. The medium composition of claim 10, wherein the antioxidant is included at a concentration of 0.001%(w/v) or more with respect to the total medium composition.

12. The medium composition of claim 9, wherein the microorganism is *Yarrowia lipolytica.*

13. The medium composition of claim 9, wherein the medium composition increases the retinol production of the microorganism.

14. The medium composition of claim 9, wherein the medium composition increase growth of the microorganism.

15. A composition for producing retinol, the composition comprising a microorganism of the genus *Yarrowia* or a culture thereof and an antioxidant.

16. Use of an antioxidant; a medium composition for a microorganism of the genus *Yarrowia* comprising the antioxidant; or a composition comprising the microorganism of the genus *Yarrowia* or a culture thereof and the antioxidant, in producing retinoids.
